# EUROPEAN PATENT APPLICATION

(11) **EP 1 489 189 A1**
(43) Date of publication of application: **22.12.2004**
(21) Application number: 03743591.4
(22) Date of filing: 04.03.2003
(51) Int. Cl.: C12Q 1/68, C12N 15/00, A61K 38/45, A61K 45/00, A61K 48/00, A61K 31/7088, A61P 35/00

(54) **DIAGNOSTICS AND REMEDIES FOR MALIGNANT BRAIN TUMOR**

(30) Priority: 04.03.2002 JP 2002057926
(71) Applicant: Institute of Gene and Brain Science, Tokyo 160-0015 (JP)
(72) Inventor: TODA, Masahiro, Keio University Medicine School, Tokyo 160-8582 (JP); KAWAKAMI, Yutaka, Keio University Medicine School, Tokyo 160-8582 (JP); UEDA, Masakazu, Keio University Medicine School, Tokyo 160-8582 (JP); OHASHI, Yohei, Keio University Medicine School, Tokyo 160-8582 (JP)
(74) Representative: Jones, Helen Marjorie Meredith
(86) International application number: PCT/JP2003/002489
(87) International publication number: WO 2003/074736

(57) **Abstract**

A diagnostic method and a diagnostic agent for cancers such as human glioma and a therapeutic method and a therapeutic agent for cancers such as human glioma are provided by identifying tumor suppressor genes and oncogenes useful for diagnoses and treatment of cancers such as human glioma. Tumor suppressor genes such as RFX1 gene and BGT-1 gene and oncogenes such as HOXD9 gene are screened by comparing the degree of methylation of cytosine residues in CpG islands between genomic DNA derived from human glioma or human glioma cell lines and that derived from normal tissue. By targeting these tumor suppressor genes or oncogenes, cancers such as human glioma are diagnosed or treated.

## Description

### TECHNICAL FIELD

The present invention relates to methods for screening for tumor suppressor genes or oncogenes in cancers such as glioma, a type of human malignant brain tumor; diagnostic methods and diagnostic agents for cancers such as human glioma; and therapeutic methods and therapeutic agents for cancers such as human glioma.

### BACKGROUND ART

The anomalies of oncogenes or tumor suppressor genes are deeply involved in cancer development and progression. Especially, it has been clarified that tumor suppressor genes such as p53 are the master genes regulating gene expression; the anomalies of tumor suppressor genes are considered to play a critical role in canceration. It has recently been reported that DNA methylation is involved in cancer development (Adv. CancerRes., 72,141-196, 1998). In addition, attention has been focused on abnormal methylation of genomic DNA as the mechanism of tumor suppressor gene inactivation. The region rich in CpG sequences located in the 5' region of a gene is called a CpG island. Many CpG sequences in human genome are usually methylated, but CpG sequences in CpG islands in normal tissues have not undergone methylation regardless of the presence or absence of gene expression. In cancer, however, CpG islands are methylated, and expression of various genes are suppressed or lost. The methylated CpG-binding protein called methyl CpG binding repressor 2 (MeCP2) binds to methylated CpG islands and recruits complexes of histone deacetylation enzymes or chromatin-remodeling factors to the methylated region. As a result, surrounding chromatin structures change into condensed structures, preventing RNA polymerases and transcription factors from entering the promoter region so that the transcription level decreases (J. Biochem., 125, 217-222, 1999).

In recent years, the restriction landmark genome scanning (RLGS) method using the methylation-sensitive restriction enzyme NotI has been developed as a technique for identifying methylated CpG islands.

An object of the present invention is to provide a diagnostic method and a diagnostic agent for cancers such as human glioma as well as a therapeutic method and a therapeutic agent for cancers such as human glioma by identifying tumor suppressor genes or oncogenes useful for diagnosis and treatment of cancers such as human glioma.

### DISCLOSURE OF INVENTION

The inventors analyzed the abnormalities of methylation in glioma using the RLGS method employing a methylation-sensitive restriction enzyme and found that the expression of the RFX 1 and BGT-1 genes in glioma is reduced or lost, that the HOXD9 gene is ectopically expressed in glioma, and so forth. As a result, the usefulness of the genes as diagnostic and therapeutic agents, which were identified by the aforementioned RLGS method, has been uncovered, and thus the present invention has been accomplished.

Thus, the present invention relates to the following: A method for screening for a tumor suppressor gene or an oncogene, including comparing the degree of methylation of cytosine residues in CpG islands between genomic DNA derived from a human glioma or a human glioma cell line and that derived from a normal tissue (claim 1); the method for screening for a tumor suppressor gene or an oncogene of claim 1, in which the tumor suppressor gene or the oncogene is the tumor suppressor gene or the oncogene in an human glioma (claim 2); the method for screening for a tumor suppressor gene or an oncogene of claim 2, in which the tumor suppressor gene in human glioma is the RFX1 gene or the BGT-1 gene (claim 3) ; the method for screening for a tumor suppressor gene or an oncogene of claim 3, in which intron 7 of the RFX1 gene is used as the RFX1 gene (claim 4) ; the method for screening for a tumor suppressor gene or an oncogene of claim 2, in which the oncogene in human glioma is a HOX gene such as HOXD1, HOXD3, HOXD4, HOXD8, HOXD9, HOXD10, HOXD13, HOXA9, HOXB9, or HOXC9 (claim 5); a method for diagnosing a cancer such as a human glioma, including measuring at least one of the degree of methylation, the presence or absence of gene mutation, the level of gene expression, and the level of protein expression of the tumor suppressor gene or the oncogene in human glioma etc., obtained by the screening method of any one of claims 1 to 5 (claim 6) ; a diagnostic agent for a cancer such as a human glioma, including a reagent that allows measurement of at least one of the degree of methylation, the presence or absence of gene mutation, the level of gene expression, and the level of protein expression of the tumor suppressor gene or the oncogene in a cancer such as a human glioma, obtained by the screening method of any one of claims 1 to 5 (claim 7); a therapeutic method for a cancer such as a human glioma, including either expressing in the cancer cell the tumor suppressor gene in a cancer such as a human glioma, obtained by the screening method of any one of claims 1 to 4, or administering to a cancer patient at least one of a gene product of the aforementioned tumor suppressor gene, a methyltransferase inhibitor, and a histone deacetylase inhibitor (claim 8); a therapeutic agent for a cancer such as a human glioma, containing at least one of the tumor suppressor gene in a cancer such as human glioma, obtained by the screening method of any one of claims 1 to 4, a gene product of the aforementioned tumor suppressor gene, a methyltransferase inhibitor, and a histone deacetylase inhibitor (claim 9); a therapeutic method for a cancer such as a human glioma, including administering to a cancer patient an expression inhibitor of the oncogene in a cancer such as a human glioma etc., obtained by the screening method of claims 1, 2, or 5, or a compound such as a peptide and a protein specifically binding to an expression inhibitor of the oncogene (claim 10); a therapeutic agent for a cancer such as a human glioma, containing an expression inhibitor of the oncogene in a cancer, such as a human glioma, obtained by the screening method of claims 1, 2, or 5, or a compound such as a peptide and a protein specifically binding to an expression inhibitor for said oncogenes (claim 11); a diagnostic or therapeutic method for a cancer such as a human glioma, comprising targeting the tumor suppressor gene or the oncogene in a cancer such as a human glioma, obtained by the screening method of any one of claims 1 to 5 (claim 12) ; a diagnostic or therapeutic method for a cancer such as a human glioma, including targeting the RFX1 gene or the HOX gene family (claim 13) ; a diagnostic or therapeutic agent for a cancer such as a human glioma, including targeting the tumor suppressor gene or the oncogene in a cancer such as a human glioma, obtained by the screening method of any one of claims 1 to 5 (claim 14); and a diagnosing or therapeutic agent for a cancer such as a human glioma, including targeting the RFX1 gene or the HOX gene family (claim 15).

### BRIEF DESCRIPTION OF THE DRAWINGS

FIG. 1A shows results of the observation of the spots in RLGS profiles. FIG. 1B shows the spots with reduced or lost signal intensity identified as a result of RLGS analysis.
FIG. 2 shows results of BLAST search with the RLGS clone.
FIG. 3 shows an analysis of the presence or absence of expression of the RFX1 gene in normal tissues, glioma tissues, and glioma cell lines by RT-PCR.
FIG. 4 shows an analysis of the presence or absence of expression of the BGT-1 gene in normal tissues, glioma tissues, and glioma cell lines by RT-PCR.
FIG. 5 shows an analysis of the presence or absence of expression of the HOXD9 gene in normal tissues, glioma tissues, and glioma cell lines by RT-PCR.
FIG. 6 shows an analysis of changes in expression of the RFX1 gene in an agent-treated human glioma cell line (U251) by RT-PCR.
FIG. 7 shows an analysis of differences in expression of the BGT-1 gene due to treatment with 5-azacytidine and trichostatin A among four kinds of glioma cell lines by RT-PCR.
FIG. 8 shows CpGs in intron 7 of the RFX1 gene.
FIG. 9 shows an analysis of CpG methylation in intron 7 of the RFX gene of the normal human brain tissue and normal human lymphocytes by bisulfite genomic sequencing.
FIG. 10 shows an analysis of CpG methylation in intron 7 of the RFX1 gene of human glioma cell lines by bisulfite genomic sequencing.
FIG. 11 shows an analysis of CpG methylation in intron 7 of the RFX1 gene of human glioma tissues by bisulfite genomic sequencing.
FIG. 12 shows an analysis of enhancer activity in intron 7 of the RFX1 gene by luciferase assay.
FIG. 13A shows a confirmation of expression of RFX1 in U251 glioma cells by western blot using anti-RFX1 antibody. FIG. 13B shows a result of analysis of cell proliferation using [³H] thymidine uptake as an index.
FIG. 14 shows an analysis of expression of the HOXD8 gene in the normal brain tissue and various glioma cell lines by RT-PCR.
FIG. 15 shows an analysis of expression of the HOXA9, HOXB9, and HOXC9 genes in the normal brain tissue and various glioma cell lines by RT-PCR.
FIG. 16 shows an analysis of expression of HOXD family genes in the normal brain tissue together with various glioma cell lines and tissues by RT-PCR.

### BEST MODE FOR CARRYING OUT THE INVENTION

The method for screening for tumor suppressor genes or oncogenes , according to the present invention is not particularly limited, as long as it is a method for comparing the degree of methylation of cytosine residues in CpG islands between genomic DNAs derived from human glioma or human glioma cell lines and those derived from normal tissues. Tumor suppressor genes that are to be the subjects of the aforementioned screening include tumor suppressor genes in human glioma etc. such as the RFX1 gene and the BGT-1 gene. Oncogenes that are to be the subjects of the aforementioned screening include oncogenes in human glioma etc. such as HOX genes (HOXD1, HOXD3, HOXD4, HOXD8, HOXD9, HOXD10, HOX D13, HOXA9, HOXB9, HOXC9, etc.). In addition, intron 7 of the RFX1 gene can be particularly advantageously used as the aforementioned RFX1 gene.

The method for investigating the methylation status of cytosine residues in CpG islands is not particularly limited, as long as it is one of methods for investigating the degree of methylation of cytosine residues in CpG islands. Such methods include the methods known in the art, such as the method using a methylation-sensitive restriction enzyme (Nucleic Acids Res., 26, 2255-2264, 1998), the method using chemical modification with hydrazine, permanganic acid, or sodium bisulfite, the immunological method using an antibody specific to methylated DNA (Nucleic Acids Res., 26, 2255-2264, and 1998) ; the affinity column method using methyl-CpG binding domain (MBD) such as MeCP2, and the denaturing gradient gel electrophoresis (DGGE) method (Proc.Natl.Acad.Sci.USA, 96, 2913-2918, 1999), and RLGS analysis using the methylation-sensitive enzyme NotI can be indicated to be preferable.

The diagnostic method for cancers such as human glioma according to the present invention is not particularly limited, as long as it is a cancer diagnostic method targeting the tumor suppressor genes or oncogenes in human glioma etc., obtained by the aforementioned screening method. One example is the method for measuring at least one of the degree of methylation, the presence or absence of gene expression, the level of gene expression, and the level of protein expression of the aforementioned tumor suppressor genes such as the RFX1 gene or oncogenes such as the HOX gene family. Especially, the RFX1 gene, preferably intron 7 of the RFX1 gene, is useful in diagnostic methods for human glioma. One example of the method for measuring the aforementioned degree of methylation is the aforementioned method for investigating the methylation status of cytosine residues in CpG islands. Examples of the method for investigating the presence or absence of gene expression include the PCR-SSCP method and a method for comparing DNA sequences. Examples of the method for measuring the level of gene expression include the RT-PCR method etc. using primers specific to the aforementioned tumor suppressor genes or oncogenes. Examples of the method for measuring the level of protein expression include western blotting etc. using a monoclonal antibody against gene products of the aforementioned tumor suppressor genes or oncogenes. Specific examples of the specimen to be used for such diagnostic methods include, but not limited to, subjects' cells, genomic DNA, RNA, cDNA or protein obtainable from, for example, blood, urine, saliva, biopsies of tissue, etc.

The diagnostic agent for cancers such as a human glioma according to the present invention is not particularly limited, as long as it is a cancer diagnostic agent targeting the tumor suppressor genes or oncogenes in human glioma etc., obtained by the aforementioned screening method. One such example is the diagnostic agent including a reagent that allows measurement of at least one of the degree of methylation, the presence or absence of gene expression, the level of gene expression, and the level of protein expression of the aforementioned tumor suppressor genes such as the RFX1 gene or oncogenes such as the HOX gene family. Especially, the RFX1 gene, preferably intron 7 of the RFX1 gene is useful in diagnostic agents for human glioma. Examples of the reagent that allows measurement of the aforementioned degree of methylation include methylation-sensitive enzymes and reagents for RLGS analysis. One example of the reagent that allows measurement of the presence or absence of gene expression is reagents for PCR-SSCP analysis. Examples of the reagent that allows measurement of the level of gene expression include primers and RT-PCR reagents specific to aforementioned tumor suppressor genes or oncogenes, etc. Examples of the reagent that allows measurement of the level of protein expression include reagents for western blotting, etc., such as monoclonal antibodies and labeled secondary antibodies, against gene products of the aforementioned tumor suppressor genes or oncogenes.

The therapeutic method for cancers such as a human glioma according to the present invention is not particularly limited, as long as it is a therapeutic method for cancers, such as human glioma, which targets the tumor suppressor genes such as the RFX1 gene and the BGT-1 gene obtained by the aforementioned screening method. In addition, a therapeutic agent for cancers such as a human glioma according to the present invention is not particularly limited, as long as it is a therapeutic method for cancers, such as human glioma, which targets the tumor suppressor genes such as the RFX1 gene and the BGT-1 gene, obtained by the aforementioned screening method. However, the aforementioned RFX1 gene is particularly useful in therapeutic methods and therapeutic agents for human glioma. One example of the aforementioned therapeutic method for cancers such as human glioma, which targets the tumor suppressor genes such as the RFX1 gene and the BGT-1 gene is, for instance, the method either for expressing the tumor suppressor genes such as the RFX1 gene and the BGT-1 gene in cancer cells, or administering to a cancer patient at least one of gene products of the aforementioned tumor suppressor genes, a methyltransferase inhibitor, and a histone deacetylase inhibitor. Further, the aforementioned therapeutic agent for cancers such as a human glioma, which targets tumor suppressor genes such as the RFX1 gene and the BGT-1 gene, is not particularly limited, as long as it contains at least one of tumor suppressor genes such as the RFX1 gene and the BGT-1 gene, preferably an expression vector harboring a tumor suppressor gene, gene products of the tumor suppressor gene, a methyltransferase inhibitor, and a histone deacetylase inhibitor.

Local administration of an expression vector harboring a tumor suppressor gene such as the aforementioned RFX1 gene or BGT-1 gene, performed as a gene therapy treatment, enables local and stable provision of gene products of a tumor suppressor gene, due to stable expression of a tumor suppressor gene, as compared with local administration of a therapeutic agent using gene products of the tumor suppressor gene as the effective ingredient. For example, introduction of a tumor suppressor gene into human glioma cells using an expression vector allows stable expression for a predetermined period of time. Such vectors preferably include virus vectors, such as herpes simplex virus (HSV) vectors, adenovirus vectors, human immunodeficiency virus (HIV) vectors, and cytomegalovirus (CMV) vectors, but among these virus vectors, HSV and CMV vectors are more preferable. Especially, HSV vectors are safe in that HSV is not incorporated into genomic DNA of cells, and it is possible to control the expression period of transgenes. Expression vectors harboring these tumor suppressor genes can be prepared using the conventional methods.

Methyltransferase inhibitors as therapeutic agent for human glioma include compounds such as 5-azacytidine, 5-aza-2'-deoxycytidine, and S-adenosylhomocysteine. Histone deacetylase inhibitors as therapeutic agent for human glioma include compounds such as butyric acid, trichostatin A (J. Biol.Chem.265, 17174-17179, 1990) and trapoxin (J. Biol.Chem.268, 22429-22435, 1993), both of which are microbial metabolites, depudecin (Proc.Natl.Acad.Sci.USA 95, 3356-3361, 1998), phenyl butyric acid (J. Natl.Cancer Inst. 908(21), 1621-1625, 1998), FR-901228 (Exp.Cell Res. 241, 126-133, 1998), MS-27-275 (Proc. Natl. Acad. Sci. USA 96, 4592-4597, 1999), benzamide derivatives (Japanese Laid-Open Application No. 11-335735), and dithiol derivatives (Japanese Laid-Open Application No. 2001-354694).

The therapeutic method for cancers such as human glioma according to another aspect of the present invention is not particularly limited, as long as it is a therapeutic method for cancers, such as human glioma, which targets the oncogenes, such as HOX genes, obtained by the aforementioned screening method. The therapeutic agent for cancers such as human glioma according to another aspect of the present invention is not particularly limited, as long as it is a therapeutic agent for cancers, such as human glioma, which targets the oncogenes such as HOX genes etc., obtained by the aforementioned screening method. One example of the therapeutic method for cancers, such as human glioma, which targets the oncogenes such as the aforementioned HOX genes is a method for administering to a cancer patient an expression inhibitor of the aforementioned oncogenes or a compound such as peptide and protein, which specifically binds to gene products of the aforementioned oncogenes. A therapeutic agent for cancers such as human glioma according to another aspect of the present invention is not particularly limited, as long as it contains a compound such as peptide and protein, which specifically binds to the expression inhibitor of the aforementioned oncogenes and gene products of the aforementioned oncogenes.

One example of the expression inhibitor of the aforementioned oncogenes is, for example, the whole or part of antisense strands of oncogene DNA or mRNA, preferably DNA or RNA consisting of 20 bp or more. For local administration of such antisense strands, a virus vector can be used as described above, but antisense strands can also be selectively introduced into glioma etc. by using monoclonal antibodies against cancer cells of human glioma etc. Examples of the compound such as peptide and protein, which specifically binds to gene products of the aforementioned oncogenes are antibodies such as monoclonal antibody against gene products of oncogenes or the variable region thereof.

Various mixing ingredients for preparation, such as a pharmaceutically acceptable common carrier, a binder, a stabilizer, an excipient, a diluent, a buffer, a disintegrator, a solubilizer, a solubilizing agent, and a tension agent, may suitably be added to the aforementioned cancer therapeutic agent according to the present invention. Such a therapeutic agent can be administered orally or parenterally. That is, it may be administered orally in commonly used administration forms such as, for example, powders, granules, capsules, syrups, and suspensions, or parenterally and locally in the form of an injection in dose forms such as a solution, an emulsion, and a suspension. The agent may also be administered in a spray form into the nostrils. The dosage of these therapeutic agents of the present invention is appropriately selected based on the direction for use, the patient's age or sex, the degree of the disease, and other conditions. Usually, the amount of active ingredient compound is preferably about 0.0001 to 100 mg per body weight of 1 1kg per day. Moreover, it is desirable that the active ingredient compound is contained in the range of 0.001 to 1, 000 mg in the preparation of the administration unit form.

The embodiments in which the cancer in the diagnostic and therapeutic methods as well as diagnostic and therapeutic agents for cancers of the present invention is human glioma, a type of malignant brain tumor, have been described. However, it is to be understood the caners as the subject of the invention are not limited to glioma and they may include cancers, besides human glioma, such as cervical cancer, epithelioid cancer, T androblastoma, promyelocytic leukemia, esophagus carcinoma, pancreatic cancer, malignant melanoma, lung cancer, cancer of oral cavity, breast cancer, bladder cancer, uterine cancer, ovarian cancer, neuroblastoma, and colon cancer.

The following examples explain the present invention in more detail, but are not to be construed to limit the technical scope of the present invention.

### EXAMPLE 1

### (MATERIALS AND METHODS)

### EXAMPLE 1A

### (CELL LINES, TUMOR)

Human glioma cell lines, T98G, U87MG, and A172 were obtained from the American Type Culture Collection (ATCC) and U251 and GI-1 from RIKEN Gene Bank. These glioma cell lines were cultured in DMEM supplemented with 10% fetal bovine serum. Human glioma tissues (GB4, GB13, GB16, GB17, GB26, and GB30) used were part of lesion excised during the surgical operations on the patients whose informed consent had been obtained. High-molecular weight genomic DNA was prepared from the human glioma tissues (GB26 and GB30), the blood of the patients, and the blood of healthy individuals whose informed consent had been obtained.

### EXAMPLE 1B

### (RLGS ANALYSIS USING METHYLATION-SENSITIVE ENZYME NOTI)

RLGS was performed basically according to the method of Matsuzaki et al. (Electrophoresis, 16, 1995). The genomic DNAs extracted from tumor tissues and bloods were digested with the methylation-sensitive enzyme NotI, and then end-labeled with ³²P. The genomic DNAs were subjected to further digestion with the restriction enzyme PvuII, followed by the first-dimension electrophoresis in agarose gel. The agarose gel was then enzyme-treated in the solution containing the restriction enzyme PstI, followed by the second-dimension electrophoresis in polyacrylamide gel. After the electrophoresis, the polyacrylamide gel was dried and the labeled DNA fragments were visualized by radioautography.

### EXAMPLE 1C

### (ISOLATION AND GENOMIC SEQUENCING OF DNA FRAGMENTS)

The RLGS profiles of DNAs derived from human glioma were compared with those of DNAs derived from the bloods obtained from the patients whose glioma tissues were used. The spots that were either reduced or lost in common between two glioma samples were excised from the RLGS gel of DNAs derived from human placenta. The DNA fragments obtained were eluted from the gel and extracted with phenol/chloroform/isoamyl alcohol. The extracted DNA fragments were ligated to NotI and PstI adapters and amplified by PCR using adapter-specific primers. Sequence reactions were performed with the Big Dye Terminator Cycle Sequencing Ready Reaction Kit (manufactured by PE Applied Biosystems), and analysis was carried out with the ABI PRISM 3100 Genetic Analyzer. When the quantity of DNA was subtle and direct sequencing was impossible, PCR product was subcloned into pGEM-T Easy vector (manufactured by Promega Corp.) prior to sequencing. The resulting sequences were analyzed for sequence homology using the database of National Center for Biotechnolology Information (NCBI).

### EXAMPLE 1D

### (RNA PREPARATION AND RT-PCR)

Total RNAs derived from human glioma tissues (GB4, GB13, GB16, and GB17) and human glioma cell lines were extracted with Trizol (manufactured by GIBCO), and total RNAs of normal tissues were purchased from Clontech. cDNAs were synthesized from 10 µg of each of total RNAs using the reverse transcriptase XL (AMV) (manufactured by Takara Shuzo Co., Ltd.) in a final volume of 10 µl. PCR was performed in the system in a total volume of of 25 µl, using 1 µl cDNA template and Takara Taq™ (manufactured by Takara Shuzo Co., Ltd.). The reaction mixture was supplemented with 2.5 µl of 10 x PCR-Buffer (100 mM Tris-HCl [pH 8.3], 500 mM KCl, 15 mM MgCl₂), 2 µl of deoxyribonucleotide triphosphate mixture (2.5 mM each), and 0.1 µM primers.

The primer sequences used were as follows: For the RFX1 gene, sense primer: 5'-GAA GAT GGA AGG CAT GAC C-3' (SEQ ID NO: 1) and antisense primer: 5'-GGC TCT TGG CAA AGT TCC-3' (SEQ ID NO: 2) ; for the HOXA9 gene, sense primer: 5'-CGA AGG CGC CTT CTC CGA AA-3'(SEQ ID NO: 3) and antisense primer: 5'-AAA TGG CAT CAC TCG TCT TTT GCT C-3' (SEQ ID NO: 4) ; for the HOXB9 gene, sense primer: 5'-CAC GCC CGA GTA CAG TTT GG-3' (SEQ ID NO: 5) and antisense primer: 5'-CAC TTG TCT CTC ACT CAG ATT GAG G-3' (SEQ ID NO: 6); for HOXC9 gene, sense primer: 5'-GGC AGC AAG CAC AAA GAG GA-3'(SEQ ID NO: 7) and antisense primer: 5'-AGG CTG GGT AGG GTT TAG GAC-3' (SEQ ID NO: 8) ; for the HOXD9 gene, sense primer: 5'-CTT GAC CCA AAC AAC CCC-3' (SEQ ID NO: 9) and antisense primer: 5'-CTC TCT GTT AGG TTG AGA ATC C-3' (SEQ ID NO: 10); for the HOXD8 gene, sense primer: 5'-GCC AGG AGT ACT TCC ACC-3' (SEQ ID NO: 11) and antisense primer: 5'-GTT TCC CCG TCC TTC ACC-3' (SEQ ID NO: 12); for the BGT-1 gene, sense primer: 5'-GAG CAT TGC ACG GAC TTT CTG AAC C-3' (SEQ ID NO: 13) and antisense primer: 5'-CCA GGA TGG AGA AGA CAA CAA ACC C-3' (SEQ ID NO: 14)]; for GAPDH, sense primer: 5'-TGA ACG GGA AGC TCA CTG G-3' (SEQ ID NO: 15) and an antisense primer: 5'-TCC ACC ACC CCC CTG TTG CTG TA-3' (SEQ ID NO: 16); for β-actin, sense primer: 5'-GTC GAC AAC GGC TCC GGC ATG TGC-3' (SEQ ID NO: 17) and antisense primer: 5'-GGA TCT TCA TGA GGT AGT CAG TCA G-3' (SEQ ID NO: 18).

PCR was performed under the following reaction conditions:
(a) For RFX1, an initial denaturation at 94°C for 5 min, followed by 30 cycles of denaturation at 94°C for 1 min, annealing at 57°C for 1 min, and extension at 72°C for 1 min, with a final extension at 72°C for 7 min.
(b) For HOXA9, B9, and C9, an initial denaturation at 94°C for 5 min, followed by 35 cycles of denaturation at 94°C for 1 min, annealing at 62°C for 1 min, and extension at 72°C for 1 min, with a final extension at 72°C for 7 min.
(c) For HOXD9, an initial denaturation at 94°C for 5 min, followed by 35 cycles of denaturation at 94°C for 1 min, annealing at 57°C for 1 min, and extension at 72°C for 1 min, with a final extension at 72°C for 7 min.
(d) For HOXD8, an initial denaturation at 94°C for 5 min, followed by 35 cycles of denaturation at 94°C for 1 min, annealing at 58°C for 1 min, and extension at 72°C for 2 min, with a final extension at 72°C for 7 min.
(e) For BGT1, an initial denaturation at 94°C for 5 min, followed by 35 cycles of denaturation at 94°C for 1 min, annealing at 65°C for 1 min, and extension at 72°C for 2 min, with a final extension at 72°C for 7 min.
(f) For GAPDH, an initial denaturation at 94°C for 5 min, followed by 25 cycles of denaturation at 94°C for 1 min, annealing at 58°C for 1 min, and extension at 72°C for 1 min, with a final extension at 72°C for 7 min.
(g) For β-actin, an initial denaturation at 94°C for 5 min, followed by 20 cycles of denaturation at 94°C for 1 min, annealing at 68°C for 1 min, and extension at 72°C for 2 min, with a final extension at 72°C for 7 min.

The resulting PCR products were electrophoresed on 2% agarose gel and analyzed by ethidium bromide staining.

Further, the expression of HOXD family genes was comprehensively analyzed. The primer sequences used were as follows: For the HOXD1 gene, sense primer: 5'-ACC CCA AGT CCG TCT CTC-3' (SEQ ID NO: 23) and antisense primer: 5'-AGC AGT TGG CTA TCT CGA TG-3'(SEQ ID NO: 24); for the HOXD3 gene, sense primer: 5'-CCA TCA GCA AGC AGA TCT TCC-3'(SEQ ID NO: 25) and antisense primer: 5'-TCT TGA TCT GGC GTT CCG T-3' (SEQ ID NO: 26); for the HOXD4 gene, sense primer: 5'-GGA TGA AGA AGG TGC ACG TGA ATT CGG-3' (SEQ ID NO: 27) and antisense primer: 5'-GGG TCC CCA CTT CTA TAA GGT CGT CA-3' (SEQ ID NO: 28); for HOXD9 gene, sense primer: 5'-CTT GAC CCA AAC AAC CCC-3' (SEQ ID NO: 9) and an antisense primer: 5'-CTC TCT GTT AGG TTG AGA ATC C-3' (SEQ ID NO: 10); for HOXD10, sense primer: 5'-CCA AGG CGG CCT TCC CGA AGA-3' (SEQ ID NO: 29) and antisense primer: 5'-TCG GCG GTT TTG AAA CCAAAT CTT GAC C-3' (SEQ ID NO: 30) ; for the HOXD13 gene, sense primer: 5'-GGC CTA CAT CTC CAT GGA GGG GTA CCA-3' (SEQ ID NO: 31) and antisense primer: 5'-GTG GCC AAC CTG GAC CAC ATC AGG AG-3' (SEQ ID NO: 32). The PCR reaction conditions were as follows: an initial denaturation at 94°C for 5 min, followed by 35 cycles of denaturation at 94°C for 1 min, annealing at each Tm value for 1 min, and extension at 72°C for 1 min, with a final extension at 72°C for 7 min. The Tm values were 60°C, 66°C, 57°C, 67°C, and 70°C for HOXD1 and D3, HOXD4, HOXD9, HOXD10, and HOXD13, respectively.

### EXAMPLE 1E

### (5-AZACYTIDINE AND TRICHOSTATIN A TREATMENT)

Human glioma cell lines were plated at a low density, incubated for 24 hours, and then 5-azacytidine (methyltransferase inhibitor) (manufactured by Sigma Chemical co.) was added at a final concentration of 500 nM. The culture was incubated for 3 days with changes of media containing 5-azacytidine of every 24 hours, and during the last 24 hours trichostatin A (histone deacetylation inhibitor) (manufactured by Wako Chemical Industries Ltd.) was added at a final concentration of 500 ng/ml. Subsequently, total RNAs were extracted, followed by cDNA synthesis. PCR was then performed and changes in gene expression were analyzed using each agent.

### EXAMPLE 1F

### (BISULFITE GENOMIC SEQUENCING)

Bisulfite conversion of 1 µg of genomic DNA was performed using the CpGenome DNA Modification Kit (manufactured by Intergen Co.). In this process, unmethylated cytosines were changed into uracils, whereas methylated cytosinse remained unchanged. The treated DNA was dissolved in 50 µl of distilled water. Using this DNA as a template, the first-round PCR was performed using primers specific to intron 7 of the RFX1 gene. The primer sequences used here were as follows: RFX1 intron 7 sense primer 1: 5'-GGT TTT GGG TTA GTT TTA ATT TTT-3' (SEQ ID NO: 19), RFX1 intron 7 antisense primer 1: 5'-TTC TCT AAA TCC TAA CCC TCT AA-3' (SEQ ID NO: 20). The PCR reaction condition was as follows: an initial denaturation at 98°C for 5 min, followed by 45 cycles of denaturation at 98°C for 1 min, annealing at 50°C for 2 min, and extension at 72°C for 2 min, with a final extension at 72°C for 7 min. Further, nested PCR was performed using the first-round PCR product. As a template, 1 µl of 25 times diluted first-round PCR product was used. The primer sequences used were as follows: RFX1 intron 7 sense primer 2: 5'-GGT GGA GGT TTG GAG TTT-3' (SEQ ID NO: 21), RFX1 intron 7 antisense primer 2: 5' -ACAAAAACAAAT ATA AAA ACA ACA-3' (SEQ ID NO: 22). The PCR condition was as follows: an initial denaturation at 98°C for 5 min, followed by 45 cycles of denaturation at 98°C for 1 min, annealing at 50°C for 1 min, and extension at 72°C for 1 min, with a final extension at 72°C for 7 min. This PCR product was subcloned into pGEM-T Easy Vector (manufactured by a Promega Corp.), followed by sequencing with T7 primer.

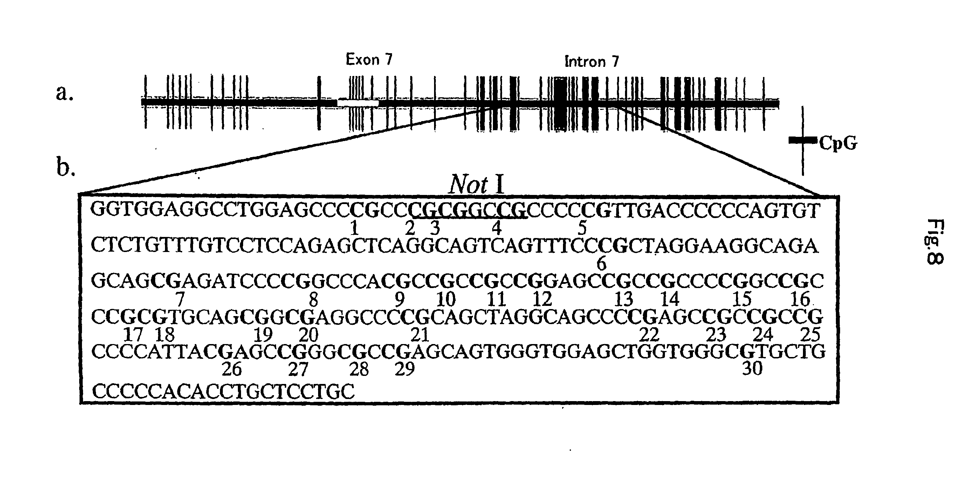

CpG methylation in intron 7 of the respective RFX1 gene of human glioma cell lines (U251, GI-1, T98G, and U87MG), human glioma tissues (4 samples), normal human brain tissues (2 samples), and normal human lymphocytes (3 samples) was analysed, using bisulfite genomic sequencing as described above. FIGS. 8 (a) and (b) show CpGs in intron 7. The same procedure described in the aforementioned bisulfite genomic sequencing was performed except the following conditions: the first-round PCR: an initial denaturation at 94°C for 5 min, followed by 45 cycles of denaturation at 94 °C for 1 min, annealing at 48 °C for 2 min, and extension at 72°C for 2 min, with a final extension at 72°C for 7 min; and the second-round PCR: an initial denaturation at 94°C for 5 min, followed by 45 cycles of denaturation at 94°C for 1 min, annealing at 50°C for 1 min, and extension at 72°C for 1 min, with a final extension at 72°C for 7 min.

### EXAMPLE 1G

### (LUCIFERASE ASSAY)

RFX1 intron 7 was isolated by PCR and incorporated into pGL3-Promoter Luciferase vector (manufactured by Promega Corp.). Primer sequences used in this PCR were as follows: sense primer: 5'-GTG TGT CTC CCC CTC CTA CCC CAC G-3' (SEQ ID NO: 33), antisense primer: 5'-GGG GCA GAG GAA GGG CAC GTG GAG G-3' (SEQ ID NO: 34). The PCR conditions were as follows: an initial denaturation at 98°C for 5 min, followed by 40 cycles of denaturation at 98°C for 1 min, annealing at 68°C for 2 min, and extension at 72°C for 2 min, with a final extension at 72°C for 7 min. Gene transfer of the resulting plasmid into U251 glioma cells was performed using LipofectAMINE PLUS™Reagent (manufactured by lnvitrogen Corp.), and after 48 hours of incubation, luciferase assay was carried out. Bright-Glo Luciferase Assay System (manufactured by Promega Corp.) was used for the assay. Bioluminescence produced by the luciferase was detected using a Wallac ARVO™ SX1420 multilabel counter (manufactured by PerkinElmer Life Sciences).

### EXAMPLE 1H

### (GLIOMA CELL PROLIFERATION INHIBITION TEST)

pFLAG-CMV-2 vector (manufactured by Sigma Corp.) was used as the vector for the gene transfer of the RFX1 gene to U251 glioma cells. The RFX1 gene was provided by Mr. W. Reith. LipofectAMINE PLUST™Reagent (manufactured by Invitrogen Corp. ) was used for the gene transfer. Glioma cell proliferation was analyzed based on the cellular thymidine uptake capacity. Specifically, 5, 000 cells, which had been subject to the gene transfer and incubated for 24 hours at 37°C in a CO₂ incubator, were transferred into each well of 96-well plates and incubated for 24 hours. Subsequently, 1 µCi of [³H] thymidine per well was added. The cells were incubated for another 24 hours and then collected. [³H] thymidine uptake was measured using the top counter.

### EXAMPLE 2

### (RESULTS)

### EXAMPLE 2A

### (RLGS PROFILES)

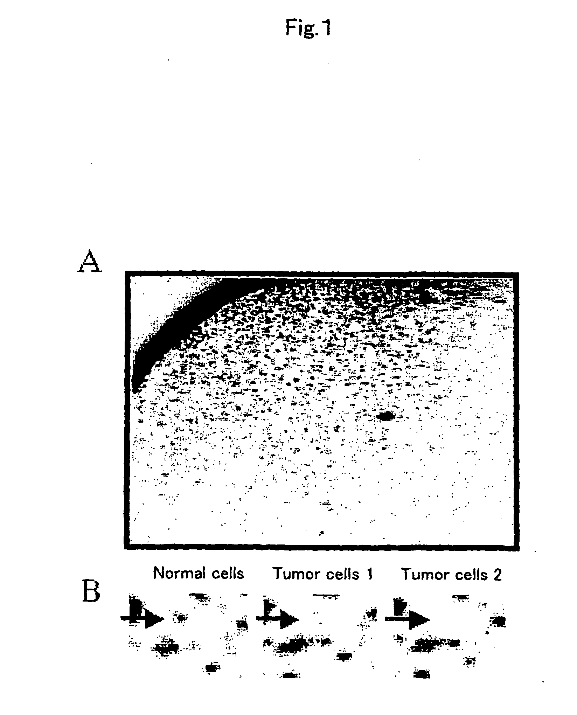

About 2, 500 spots are observed on the RLGS profiles, as shown in FIG. 1A. In this study, about 400 spots in the central part with the highest resolution were compared and analyzed between two glioma samples (GB26 and GB30) and normal samples (bloods from the corresponding patients to GB26 and GB30). In this analysis, since RLGS was performed with NotI that does not cut methylated DNA as a landmark, the spots corresponding to the abnormally methylated DNA regions were either lost or reduced in signal strength, as compared with normal samples. As a result of this analysis, 12 spots that had been lost and reduced in signal strength, in common to two glioma samples, were identified (FIG. 1B and Table 1).

**Table 1**

| Clone No. | Chromosome | GC% | CpG ratio | Related gene | Location | Gene function |
|---|---|---|---|---|---|---|
| 1 | 19 | 65 | 0.53 | RFX1 | Intron | Transcriptional regulation |
| 2 | 15 | 54.4 | 0.77 | - | | |
| 3 | 12 | 60.9 | 0.59 | BGT-1 | 3' | Neurotransmitter transporter |
| 4 | | | | Unidentified | | |
| 5 | 5 | 63.8 | 0.67 | ADAMTS2 | Intron | Protease |
| 6 | 2 | 64.6 | 0.94 | HOXD9 | 5' | Transcription factor |
| 7 | 9 | 63.8 | 0.59 | - | | |
| 8 | 15 | 67.8 | 0.59 | FKSG88 | Exon & intron | Unknown |
| 9 | 14 | 58.3 | 0.78 | CGI-112 protein | 5' | Unknown |
| 10 | X | 67.3 | 0.61 | - | | |
| 11 | 12 | 67.1 | 0.58 | ALK-1 | 3' | Intracellular signaling transducer |
| 12 | 2 | 64.4 | 0.91 | - | | |

### EXAMPLE 2B

### (ISOLATION AND IDENTIFICATION OF DNA FRAGMENTS METHYLATED IN GLIOMA)

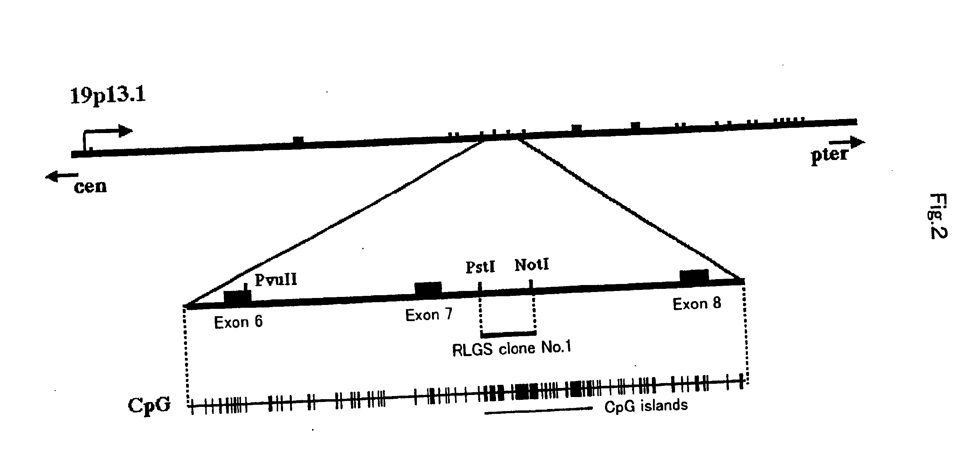

The 12 spots that had been lost in the glioma RLGS profiles were isolated from the placental RLGS trapper gel and subject to sequencing. As a result, all the analyzable clones (11) were revealed to correspond to CpG islands from the results of %GC and CpG ratio {(number of CpGs) / (number of guanine) (number of cytosines)} x (number of nucleotides analyzed) (Table 1). Further, to search for genes near these CpG islands isolated, BLAST search was performed and seven genes were identified. For example, the DNA fragment of clone 1 was revealed to be present inside intron 7 of the RFX1 gene of chromosome 19 (FIG. 2).

### EXAMPLE 2C

### (GENE EXPRESSION ANALYSIS BY RT-PCR)

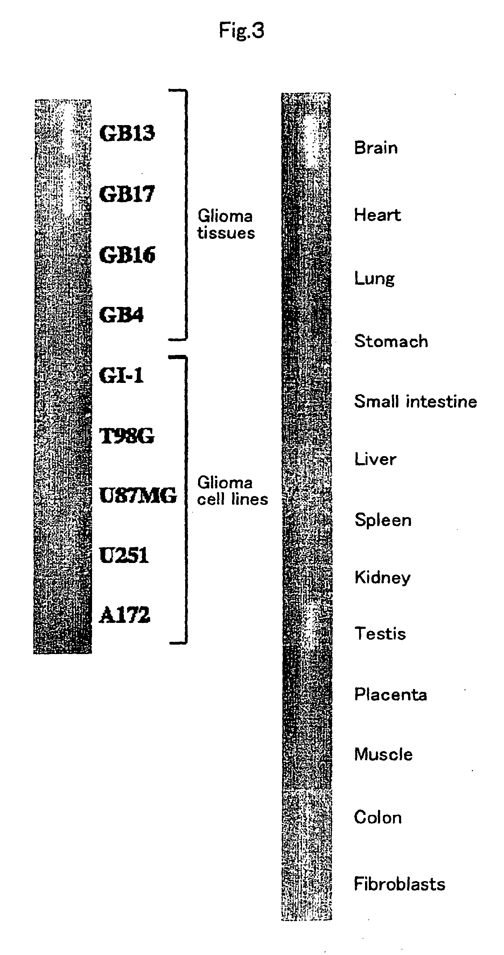

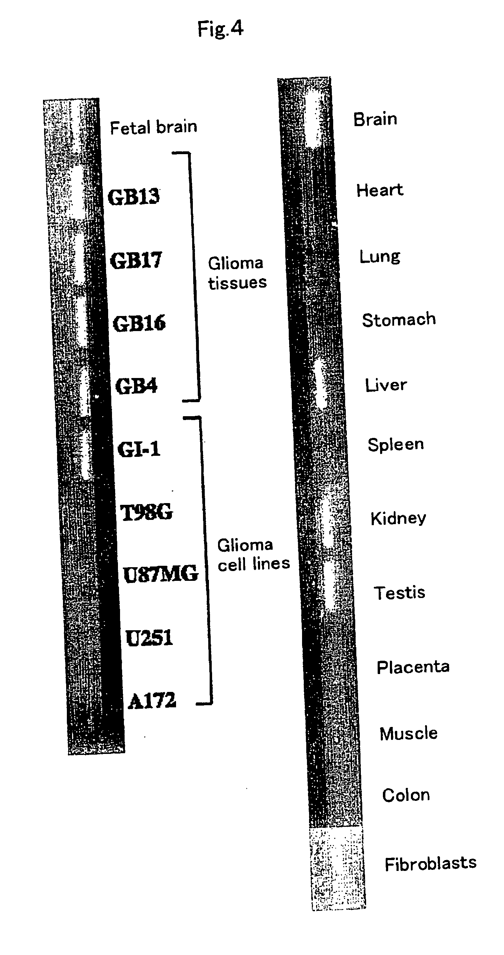

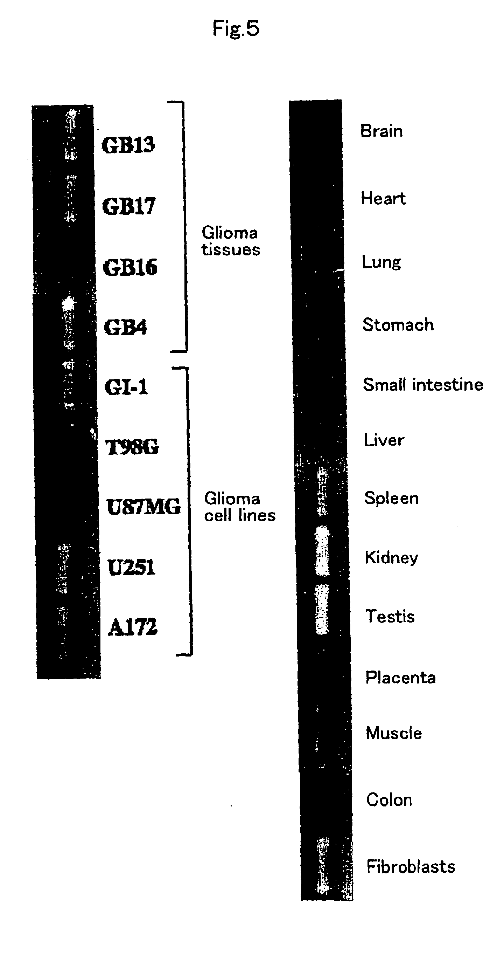

Expression of the seven genes identified in normal tissues, glioma tissues, and glioma cell lines was analyzed by RT-PCR. As a result, the following three genes exhibited interesting expression patterns. Expression of the RFX1 gene was observed in the brain and testis among normal tissues, whereas loss or reduction of its expression was observed in glioma tissues (2/4) and glioma cell lines (5/5) (FIG. 3). Likewise, expression of the BGT-1 gene was observed in the brain, liver, kidney, and testis among normal tissues, whereas loss or reduction of its expression was observed in glioma tissues (3/4) and glioma cell lines (5/5) (FIG. 4). On the other hand, expression of the HOXD9 gene was observed mainly in the spleen, kidney, and testis, but not in the brain among normal tissues, whereas its expression was observed in glioma tissues (3/4) and glioma cell lines (3/5) (FIG. 5).

### EXAMPLE 2D

### (GENE RE-EXPRESSION BY 5-AZACYTIDINE AND TRICHOSTATIN A TREATMENT)

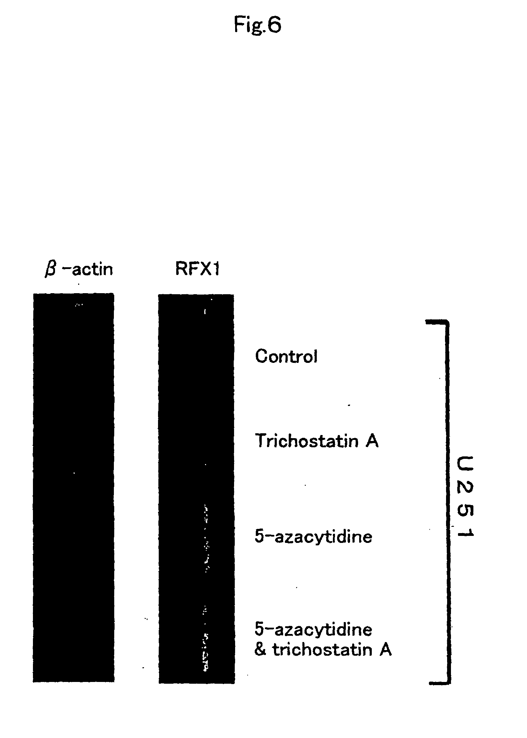

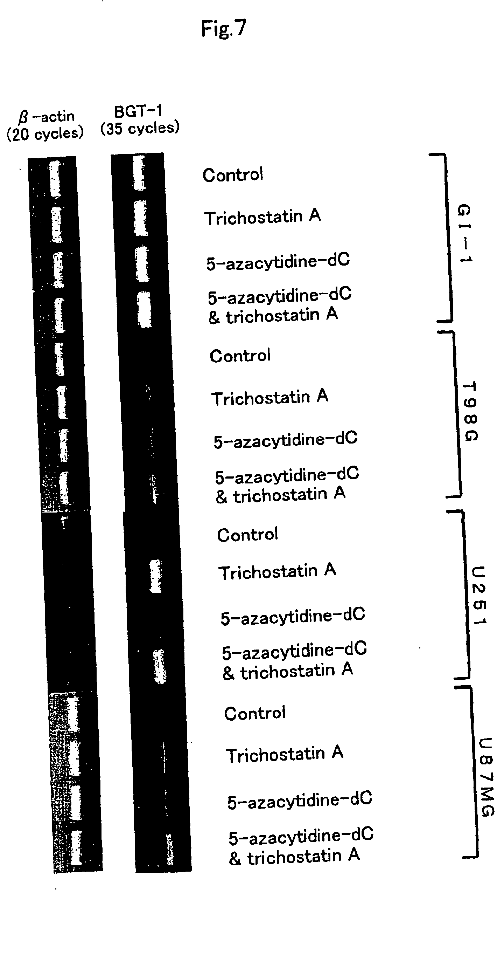

To investigate the relationship between reduction or loss of expression of the RFX1 and BGT-1 genes in glioma and DNA methylation or histone deacetylation, change in expression of each gene was analyzed using the RT-PCR method, either after treatment of human glioma cell lines with the methyltransferase inhibitor 5-azacytidine and the histone deacetylase inhibitor trichostatin A singly or after treatment of cells with both inhibitors. As a result, in human glioma U251 cells, as compared with the control, enhancement of expression of the RFX1 gene by treatment of each inhibitor was observed and further enhancement of expression by treatment of both inhibitors was observed (FIG. 6). Likewise, the BGT-1 gene also exhibited enhancement of expression by treatment of inhibitor (s) in four glioma cell lines with variations in degree, compared with the control, (FIG. 7). These results indicated that decrease in expression of RFX1 and BGT-1 in glioma is related to DNA methylation.

### EXAMPLE 2E

### (METHYLATION ANALYSIS OF INTRON 7 OF THE RFX1 GENE)

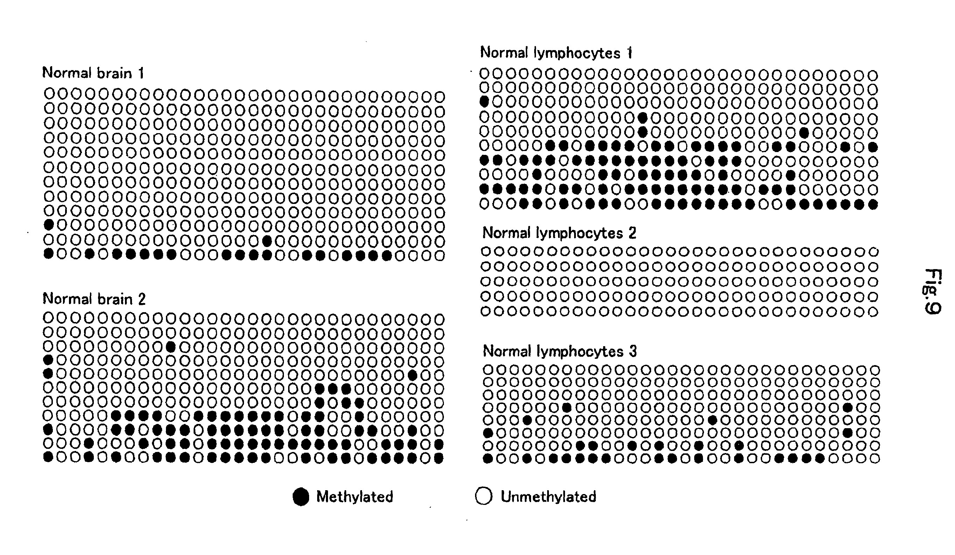

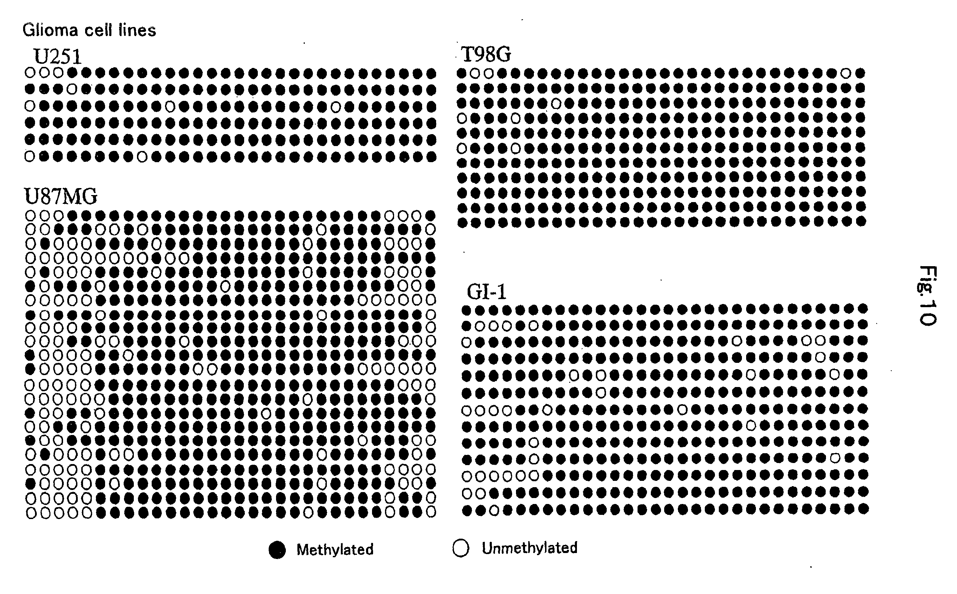

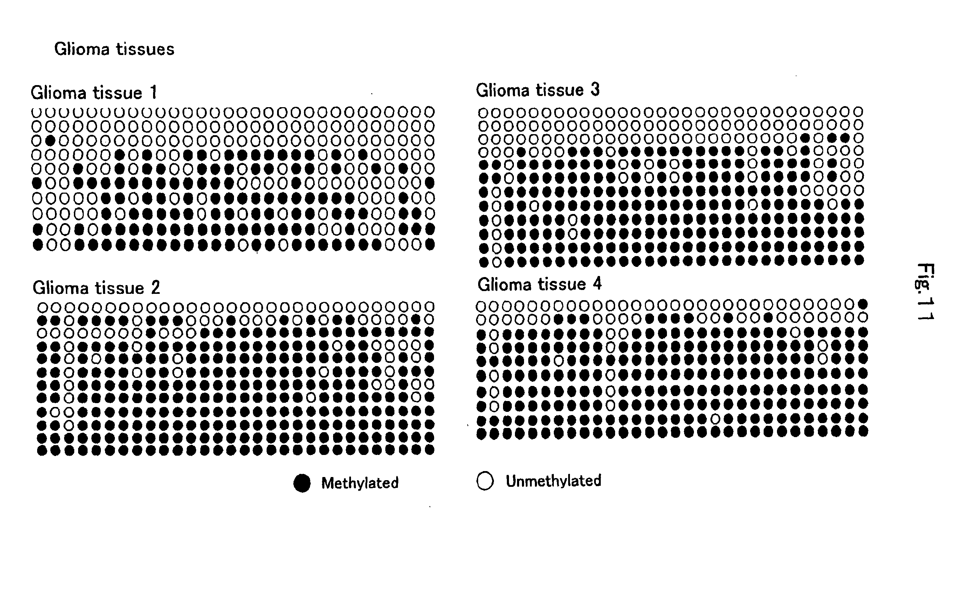

To further investigate the CpG methylation status in RFX1 intron 7 in detail, bisulfite sequencing was performed. FIG. 9, FIG. 10, and FIG. 11 show the results of CpG methylation analysis of intron 7 of the RFX1 gene in normal human brain tissues (2 samples) and normal human lymphocytes (3 samples), in human glioma cell lines (U251, GI-1, T98G, and U87MG), and in human glioma tissues (4 samples), respectively. In FIGS. 9 to 11, the black circle and the white circle show methylation and unmethylation, respectively. As a result, as shown in FIGS. 9 to 11, a high frequency of methylation was observed in CpGs of intron 7 of the RFX1 gene in glioma cell lines and tissues, as compared with normal brain tissues or lymphocytes. These results showed that analysis of methylation status of intron 7 of the RFX1 gene enables diagnosis of glioma.

### EXAMPLE 2F

### (ANALYSIS OF THE ENHANCER ACTIVITY OF INTRON 7 OF the RFX1 GENE)

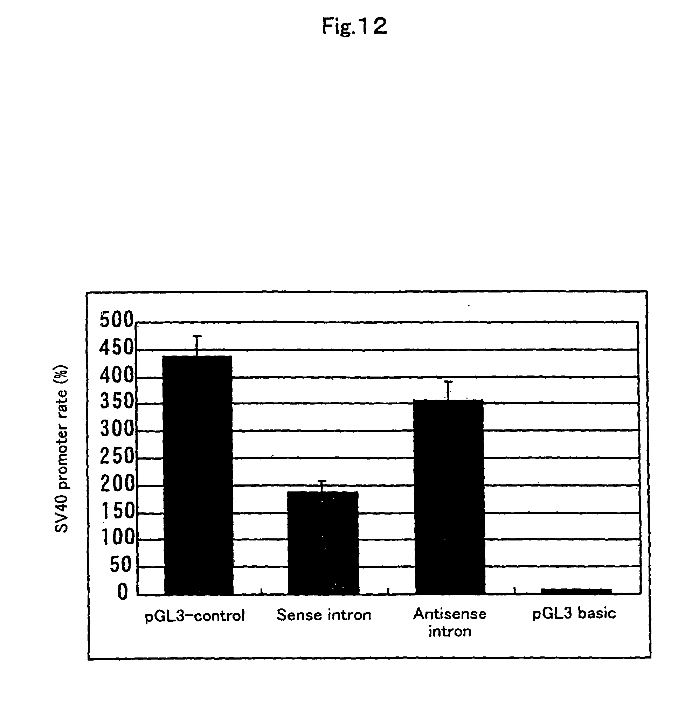

The above-described results of methylation analysis of intron 7 of the RFX1 gene suggested that methylation of intron 7 of the RFX1 gene regulates expression of the RFX1 gene. Thus, enhancer activity of RFX1 intron 7 was analyzed by luciferase assay. The results are shown in FIG. 12. The data shown in FIG. 12 is the representation of the respective values divided by the value of pGL3 promoter (a vector into which SV40 promoter was incorporated) that was used as a control. In FIG. 12, pGL3 control indicates the vector into which SV40 promoter and SV40 enhancer are incorporated, sense intron indicates the vector into which SV40 promoter and intron 7 of the RFX1 gene in the sense orientation are incorporated, antisense intron indicates the vector into which SV40 promoter and intron 7 of the RFX1 gene in the antisense orientation are incorporated, and pGL3 basic indicates the vector into which no promoter or enhancer is incorporated. As shown in FIG. 12, the vectors into which intron 7 of the RFX1 gene was incorporated, either in the sense or antisense orientation, were found to exhibit a higher luciferase luminescence than those into which SV40 promoter was independently incorporated. This revealed that intron 7 of the RFX1 gene has an enhancer activity.

### EXAMPLE 2G

### (SUPPRESSION OF GLIOMA CELL PROLIFERATION BY RFX1)

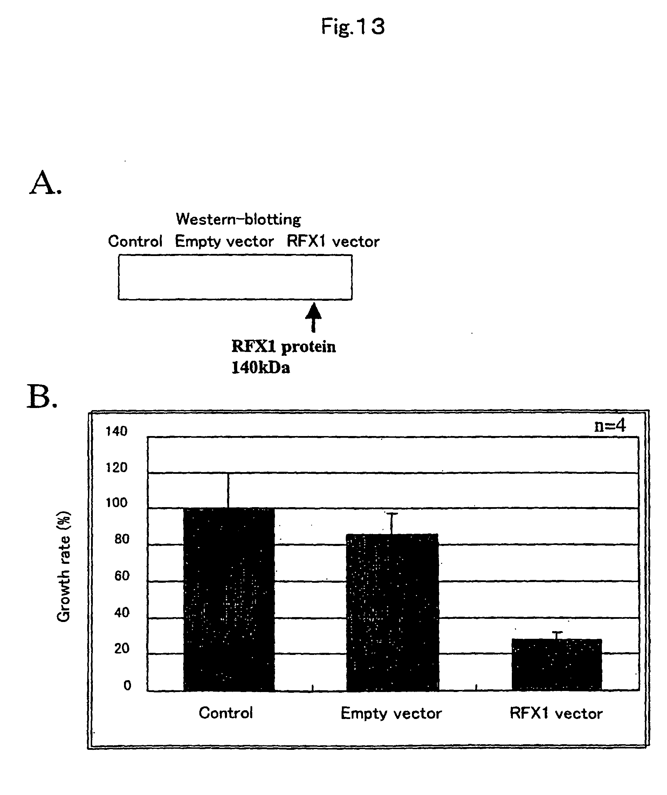

To examine the relationship between expression of RFX1 and tumor cell proliferation, gene transfer of the RFX1 gene into the U251 glioma cells was performed. First, the expression of RFX1 was confirmed by western blotting using anti-RFX1 antibody (manufactured by Sauta Cruz Biotechnology, Inc.). As shown FIG. 13A, expression of the RFX1 gene was not observed in the control, which was U251 glioma cells into which nothing was introduced, and the cells into which the vector pFLAG-CMV-2 alone was introduced (empty vector). Expression of the RFX1 gene was observed only in the cells (RFX1 vector) into which the RFX1 gene was introduced. FIG. 13B shows the results of analysis of cell proliferation using the amount of [³H] thymidine uptake as an index. As a result, the cells into which the RFX1 gene was introduced showed significant suppression of tumor cell proliferation, as compared with the control, the U251 glioma cells into which nothing was introduced, and the cells into which the vector pFLAG-CMV-2 alone was introduced. These results revealed that the RFX1 gene functions as a tumor suppressor gene in glioma. In addition, the fact that RFX1 is known to suppress expression of the c-myc gene, an oncogene, as a transcription factor also suggests that the RFX1 gene is involved in glioma development as a tumor suppressor gene. In conclusion, it is considered that the RFX1 gene is useful as a diagnostic agent by analyzing methylation status, gene expression, and protein expression. It is also considered that the RFX1 gene is useful as a therapeutic agent as well by expressing in glioma the RFX1 gene whose expression is either lost or reduced in glioma.

### EXAMPLE 2H

### (EXPRESSION ANALYSIS OF HOX GENES)

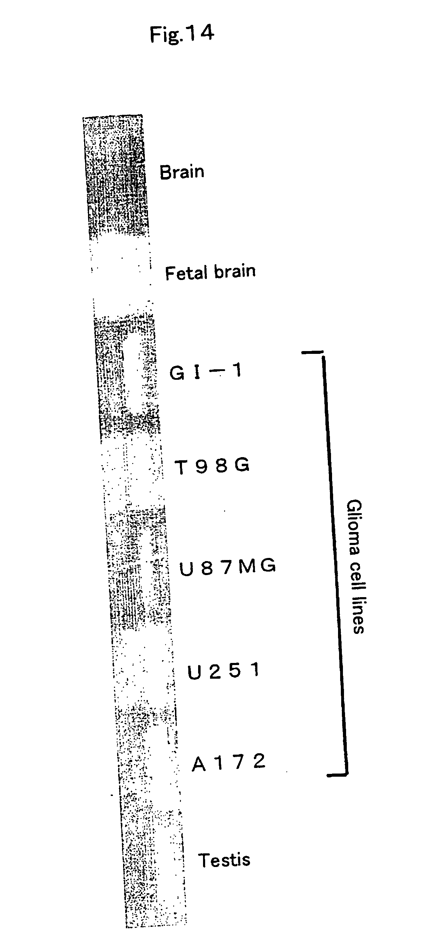

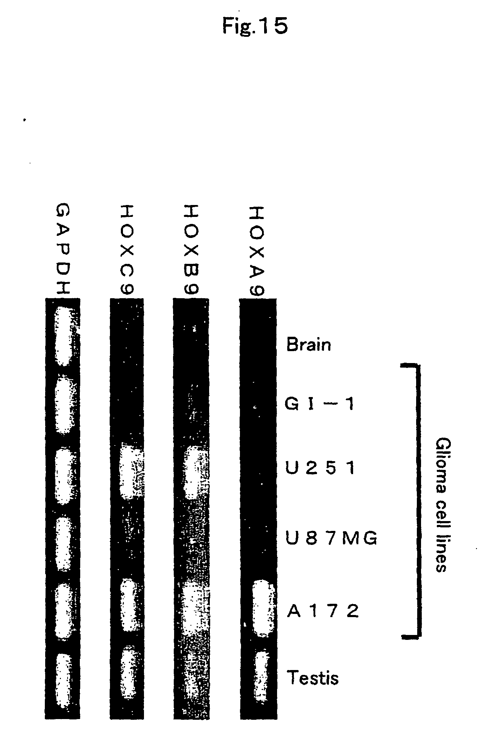

The HOX gene family functions in development of animals, determining the specificity of differentiation in the antero-posterior axis of an individual. These genes in this family have a DNA-binding sequence called homeobox, and their expression is highly regulated. As shown in FIG. 5, it was clarified that the HOXD9 gene is ectopically expressed in glioma. Further, it was analyzed whether or not the other HOX genes (HOXD8, HOXA9, HOXB9, and HOXC9), which are not intrinsically expressed in the normal brain tissue, are ectopically expressed in glioma using the RT-PCR method. As a result, none of the aforementioned HOX genes investigated (HOXD8, HOXA9, HOXB9, and HOXC9) was observed to be expressed in the brain of healthy humans, whereas gene expression was observed in glioma cell lines (FIGS. 14 and 15). It was therefore suggested that these HOX genes are involved in glioma (cancer) development as oncogenes, as a result of being methylated. In conclusion, it is considered that ectopically-expressed HOX-family genes are useful as diagnostic or therapeutic agents for cancers, including glioma.

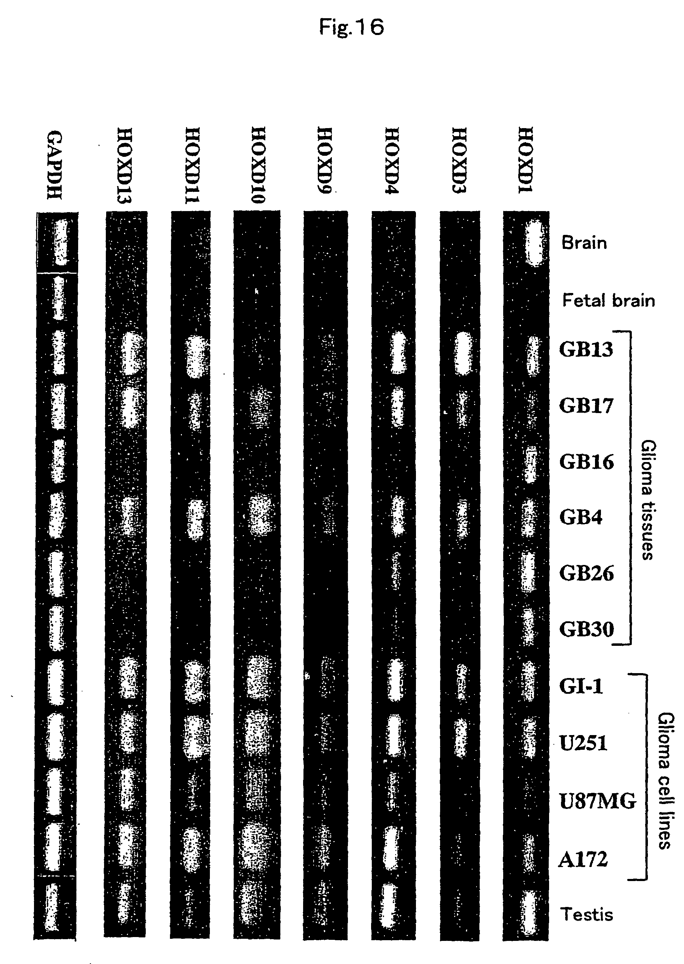

Next, FIG. 16 shows the result of extensive analysis of expression of HOXD-family genes in glioma. The results revealed that not only HOXD1, which is intrinsically expressed in the brain, but also the other HOXD-family genes (HOXD3, HOXD4, HOXD9, HOXD10, HOXD11, and HOXD13), which are not expressed in the brain, are highly expressed in brain tumors. It was therefore suggested that these HOXD-family genes, except for HOXD1, are involved in glioma (cancer) development as oncogenes as a result of being methylated. In conclusion, it is considered that ectopically-expressed HOXD-family genes are particularly useful as diagnostic and therapeutic agents for cancers, including glioma.

### INDUSTRIAL APPLICABILITY

According to the present invention, tumor suppressor genes such as the RFX1 gene and the BGT-1 gene or oncogenes such as HOX genes, which are useful for the diagnosis and treatment of cancers, such as a human glioma, can be identified. Consequently, a diagnostic method and a diagnostic agent for cancers such as human glioma as well as a therapeutic method and a therapeutic agent for cancers such as human glioma, which target the aforementioned tumor suppressor genes and oncogenes, can be provided.

## Claims

1. A method for screening for a tumor suppressor gene or an oncogene, comprising comparing the degree of methylation of cytosine residues in a CpG island between genomic DNA derived from a human glioma or a human glioma cell line and that derived from a normal tissue.

2. The method for screening for a tumor suppressor gene or an oncogenes of claim 1, wherein the tumor suppressor gene or the oncogene is the tumor suppressor gene or the oncogene in a human glioma.

3. The method for screening for a tumor suppressor gene or an oncogene of claim 2, wherein the tumor suppressor gene in the human glioma is the RFX1 gene or the BGT-1 gene.

4. The method for screening for a tumor suppressor gene or an oncogene of claim 3, wherein intron 7 of the RFX1 gene is used as the RFX1 gene.

5. The method for screening for a tumor suppressor gene or an oncogene of claim 2, wherein the oncogene in the human glioma is a HOX gene such as HOXD1, HOXD3, HOXD4, HOXD8, HOXD9, HOXD10, HOXD13, HOXA9, HOXB9, or HOXC9.

6. A method for diagnosing a cancer such as a human glioma, comprising measuring at least one of the degree of methylation, the presence or absence of gene mutation, the level of gene expression, and the level of protein expression of the tumor suppressor gene or the oncogene in the human glioma etc., obtained by the screening method of any one of claims 1 to 5.

7. A diagnostic agent for a cancer such as a human glioma, comprising an reagent that allows measurement of at least one of the degree of methylation, the presence or absence of gene mutation, the level of gene expression, and the level of protein expression of the tumor suppressor gene or the oncogene in the human glioma, obtained by the screening method of any one of claims 1 to 5.

8. A therapeutic method for a cancer such as a human glioma, comprising either expressing in a cancer cell the tumor suppressor gene in a cancer such as a human glioma, obtained by the screening method of any one of claims 1 to 4, or administering to a cancer patient at least one of a gene product of said tumor suppressor gene, a methyltransferase inhibitor, and a histone deacetylase inhibitor.

9. A therapeutic agent for a cancer such as a human glioma, containing at least one of the tumor suppressor gene in the cancer such as a human glioma, obtained by the screening method of any one of claims 1 to 4, a gene product of said tumor suppressor gene, a methyltransferase inhibitor, and a histone deacetylase inhibitor.

10. A therapeutic method for a cancer such as a human glioma, comprising administering to a cancer patient an expression inhibitor of the oncogene in the cancer of a human glioma etc. , obtained by the screening method of claims 1, 2, or 5, or a compound such as peptide and protein specifically binding to an expression inhibitor of the oncogene or gene product of the oncogene.

11. A therapeutic agent for a cancer such as a human glioma, containing an expression inhibitor of the oncogene in the cancer such as a human glioma, obtained by the screening method of claims 1, 2, or 5, or a compound such as a peptide and a protein specifically binding to an expression inhibitor for said oncogene.

12. A diagnostic or therapeutic method for a cancer such as a human glioma, comprising targeting the tumor suppressor gene or the oncogene in the cancer such as a human glioma, obtained by the screening method of any one of claims 1 to 5.

13. A diagnostic or therapeutic method for a cancer such as a human glioma, comprising targeting the RFX1 gene or the HOX gene family.

14. A diagnostic or therapeutic agent for a cancer such as a human glioma, comprising targeting the tumor suppressor gene or the oncogene in cancers such as human glioma, obtained by the screening method of any one of claims 1 to 5.

15. A diagnosing or therapeutic agent for a cancer such as a human glioma, comprising targeting the RFX1 gene or the HOX gene family.


SEQUENCE LISTING

<110> INSTITUTE OF GENE AND BRAIN SCIENCE

<120> Diagnostic and therapeutic pharmaceutical for malignant
      brain cancer

<130> HMJ03893EP

<140> 03743591.4
<141> 2003-03-04

<150> JP P2002-57926
<151> 2002-03-04

<160> 34

<170> PatentIn Ver. 2.1

<210> 1
<211> 19
<212> DNA
<213> Artificial Sequence

<220>
<223> Description of Artificial Sequence:RFX1 sense primer

<400> 1
gaagatggaa ggcatgacc                                              19


<210> 2
<211> 18
<212> DNA
<213> Artificial Sequence

<220>
<223> Description of Artificial Sequence:RFX1 antisense
      primer

<400> 2
ggctcttggc aaagttcc                                               18


<210> 3
<211> 20
<212> DNA
<213> Artificial Sequence

<220>
<223> Description of Artificial Sequence:HOXA9 sense primer

<400> 3
cgaaggcgcc ttctccgaaa                                             20


<210> 4

<211> 25
<212> DNA
<213> Artificial Sequence

<220>
<223> Description of Artificial Sequence:HOXA9 antisense
      primer

<400> 4
aaatggcatc actcgtcttt tgctc                                          25


<210> 5
<211> 20
<212> DNA
<213> Artificial Sequence

<220>
<223> Description of Artificial Sequence:HOXB9 sense primer

<400> 5
cacgcccgag tacagtttgg                                                20


<210> 6
<211> 25
<212> DNA
<213> Artificial Sequence

<220>
<223> Description of Artificial Sequence:HOXB9 antisense
      primer

<400> 6
gacttgtctc tcactcagat tgagg                                          25


<210> 7
<211> 20
<212> DNA
<213> Artificial Sequence

<220>
<223> Description of Artificial Sequence:HOXC9 sense primer

<400> 7
ggcagcaagc acaaagagga                                                20


<210> 8
<211> 21
<212> DNA
<213> Artificial Sequence

<220>
<223> Description of Artificial Sequence:HOXC9 antisense
      primer

<400> 8
aggctgggta gggtttaggac                                                21

<210> 9
<211> 18
<212> DNA
<213> Artificial Sequence

<220>
<223> Description of Artificial Sequence:HOXD9 sense primer

<400> 9
cttgacccaa acaacccc                                                   18

<210> 10
<211> 22
<212> DNA
<213> Artificial Sequence

<220>
<223> Description of Artificial Sequence:HOXD9 antisense
      primer

<400> 10
ctctctgtta ggttgagaat cc                                              22

<210> 11
<211> 18
<212> DNA
<213> Artificial Sequence

<220>
<223> Description of Artificial Sequence:HOXD8 sense primer

<400> 11
gccaggagta cttccacc                                                   18

<210> 12
<211> 18
<212> DNA
<213> Artificial Sequence

<220>
<223> Description of Artificial Sequence:HOXD8 antisense
      primer

<400> 12
gtttccccgt ccttcacc                                                   18

<210> 13
<211> 25

<212> DNA
<213> Artificial Sequence

<220>
<223> Description of Artificial Sequence:BGT-1 sense primer

<400> 13
gagcattgca cggactttct gaacc                                          25


<210> 14
<211> 25
<212> DNA
<213> Artificial Sequence

<220>
<223> Description of Artificial Sequence:BGT-1 antisense
      primer

<400> 14
ccaggatgga gaagacaaca aaccc                                          25


<210> 15
<211> 19
<212> DNA
<213> Artificial Sequence

<220>
<223> Description of Artificial Sequence:GAPDH sense primer

<400> 15
tgaacgggaa gctcactgg                                                 19


<210> 16
<211> 20
<212> DNA
<213> Artificial Sequence

<220>
<223> Description of Artificial Sequence:GAPDH antisense primer

<400> 16
tccaccaccc tgttgctgta                                                20


<210> 17
<211> 24
<212> DNA
<213> Artificial Sequence

<220>
<223> Description of Artificial Sequence:beta-actin sense primer

<400> 17
gtcgacaacg gctccggcat gtgc                                           24

<210> 18
<211> 25
<212> DNA
<213> Artificial Sequence

<220>
<223> Description of Artificial Sequence:beta-actin antisense
      primer

<400> 18
ggatcttcat gaggtagtca gtcag                                          25


<210> 19
<211> 24
<212> DNA
<213> Artificial Sequence

<220>
<223> Description of Artificial Sequence:RFX-1 7th intron sense primer

<400> 19
ggttttgggt tagttttaat tttt                                           24


<210> 20
<211> 23
<212> DNA
<213> Artificial Sequence

<220>
<223> Description of Artificial Sequence:RFX-1 7th intron antisense
      primer

<400> 20
ttctctaaat cctaaccctc taa                                            23


<210> 21
<211> 18
<212> DNA
<213> Artificial Sequence

<220>
<223> Description of Artificial Sequence:RFX-1 7th intron sense primer

<400> 21
ggtggaggtt tggagttt                                                  18


<210> 22
<211> 24
<212> DNA
<213> Artificial Sequence

<220>
<223> Description of Artificial Sequence:RFX-1 7th intron antisense
      primer

<400> 22
acaaaaacaa atataaaaac aaca                                              24


<210> 23
<211> 18
<212> DNA
<213> Artificial Sequence

<220>
<223> Description of Artificial Sequence:HOXD1 sense
      primer

<400> 23
accccaagtc cgtctctc                                                     18


<210> 24
<211> 20
<212> DNA
<213> Artificial Sequence

<220>
<223> Description of Artificial Sequence:HOXD1 antisense
      primer

<400> 24
agcagttggc tatctcgatg                                                   20


<210> 25
<211> 21
<212> DNA
<213> Artificial Sequence

<220>
<223> Description of Artificial Sequence:HOXD3 sense
      primer

<400> 25
ccatcagcaa gcagatcttc c                                                 21


<210> 26
<211> 19
<212> DNA
<213> Artificial Sequence

<220>
<223> Description of Artificial Sequence:HOXD3 antisense
      primer

<400> 26

tcttgatctg gcgttccgt                                                    19

<210> 27
<211> 27
<212> DNA
<213> Artificial Sequence

<220>
<223> Description of Artificial Sequence:HOXD4 sense
      primer

<400> 27
ggatgaagaa ggtgcacgtg aattcgg                                           27

<210> 28
<211> 26
<212> DNA
<213> Artificial Sequence

<220>
<223> Description of Artificial Sequence:HOXD4 antisense
      primer

<400> 28
gggtccccac ttctataagg tcgtca                                            26

<210> 29
<211> 21
<212> DNA
<213> Artificial Sequence

<220>
<223> Description of Artificial Sequence:HOXD10 sense
      primer

<400> 29
ccaaggcggc cttcccgaag a                                                 21

<210> 30
<211> 28
<212> DNA
<213> Artificial Sequence

<220>
<223> Description of Artificial Sequence:HOXD10
      antisense primer

<400> 30
tcggcggttt tgaaaccaaa tcttgacc                                          28

<210> 31
<211> 27

<212> DNA
<213> Artificial Sequence

<220>
<223> Description of Artificial Sequence:HOXD13 sense
      primer

<400> 31
ggcctacatc tccatggagg ggtacca                                          27


<210> 32
<211> 26
<212> DNA
<213> Artificial Sequence

<220>
<223> Description of Artificial Sequence:HOXD13
      antisense primer

<400> 32
gtggccaacc tggaccacat caggag                                           26


<210> 33
<211> 25
<212> DNA
<213> Artificial Sequence

<220>
<223> Description of Artificial Sequence:RFX1 7th intron sense
      primer

<400> 33
gtgtgtctcc ccctcctacc ccacg                                            25


<210> 34
<211> 24
<212> DNA
<213> Artificial Sequence

<220>
<223> Description of Artificial Sequence:RFX1 7th intron antisense
      primer

<400> 34
ggggcagagg aagggcacgt ggagg                                            25


,P

,P[?]^,Q